Europäisches Patentamt

European Patent Office

Office européen des brevets

(19)

(11) Publication number: **0 506 523 A2**

(12) **EUROPEAN PATENT APPLICATION**

(21) Application number: **92400738.8**

(22) Date of filing: **19.03.92**

(51) Int. Cl.⁵: **C12P 21/08**, G01N 33/577, G01N 33/92

(30) Priority: **25.03.91 JP 60259/91**

(43) Date of publication of application:
**30.09.92 Bulletin 92/40**

(84) Designated Contracting States:
**AT BE CH DE DK ES FR GB GR IT LI LU MC NL PT SE**

(71) Applicant: **DAIICHI PURE CHEMICALS CO. LTD.**
**13-5, Nihonbashi 3-chome**
**Chuo-ku Tokyo (JP)**

(72) Inventor: **Manabe, Mitsuhisa**
**2-5-26-415, Shibamata**
**Katsushika-ku, Tokyo (JP)**
Inventor: **Imoto, Kazuhiko**
**6-17-27-105, Tennohdai**
**Abiko-shi, Chiba (JP)**

(74) Representative: **Orès, Bernard et al**
**Cabinet ORES 6, Avenue de Messine**
**F-75008 Paris (FR)**

(54) **Monoclonal antibodies.**

(57) Monoclonal antibodies which are reactive with human lipoprotein (a) and do not exhibit an immune cross-reactivity with human plasminogen and apoprotein B. Human lipoprotein (a) in clinical samples can be quantitatively analyzed by the immuno-turbidimetric analysis in which at least one of the monoclonal antibody is used.

FIG. 1

① ② ③ ④ ⑤ ⑥ ⑦ ⑧ ⑨

①LDL 300mg/dl
②LDL 150mg/dl
③PLG 50mg/dl
④PLG 20mg/dl
⑤Serum No.1
⑥Serum No.2
⑦Serum No.3
⑧Serum No.4
⑨Pool Serum

EP 0 506 523 A2

Jouve, 18, rue Saint-Denis, 75001 PARIS

## BACKGROUND OF THE INVENTION

Field of the Invention:

The present invention relates to monoclonal antibodies which are useful for the immuno-turbidimetric quantitative analysis of human lipoprotein (a).

Description of the Background Art:

Serum cholesterol rich lipoproteins are known in recent years as a risk factor for causing arteriosclerotic diseases including myocardial infarction. Among these, lipoprotein (a) is a very important risk factor causing arteriosclerosis. A number of studies are ongoing about lipoprotein (a).

Lipoprotein (a) contains apoprotein (a) as a specific apoprotein having a protein structure characteristic to blood clotting or fibrinolytic system proteins in its structure. It is known to exhibit an activity of interfering the fibrinolytic system when clots are produced. Measurement of lipoprotein (a) in blood therefore is clinically very important for detecting arteriosclerotic diseases.

Immunoassay is conventionally known as a method characteristic to the measurement of lipoproteins in blood. In particular, immuno-turbidimetric quantitative analysis is a popular method in routine analyses because it is suitable for automatic analysis by autoanalyzers and ensures analysis of a large number of samples. In the immuno-turbidimetric quantitative analysis, lipoprotein (a) is insolubilized by simultaneous couplings of many antibodies at two or more antigen-determinant sites in the protein for cross-linking, thus rendering itself to be immuno-turbidimetrically analyzed. The immuno-turbidimetric quantitative analysis, therefore, are generally known to be applicable to common polyclonal antibodies. In order to obtain polyclonal antibodies, however, it is necessary to purify antigens each time they are obtained. Furthermore, the reactivity of polyclonal antibodies obtained varies depending on the individual immune animals. Constantly accurate determinations of lipoproteins in blood which are important for the pathological evaluation was difficult.

Regarding apoprotein (a), its amino acid structure is very similar to plasminogen which is a type of proteins contained in human blood at a comparatively high concentration. Therefore, even if anti-serum is obtained from animals commonly used in experiments using highly purified apoprotein (a) as an immune source, the anti-serum inevitably exhibits a cross-reactivity with plasminogen, thus making it impossible to accurately determine the amount of lipoprotein (a) by the assay system in which such an anti-serum is used.

An immune absorption method by which compounds causing the cross-reaction is reacted with the anti-serum in advance to remove the unnecessary reactivity from the anti-serum is known in the art as a method of obtaining anti-serum having only a desirable reactivity from such an anti-serum exhibiting a cross-reactivity. However, since the apoprotein (a) which is a characteristic component of lipoprotein (a) and plasminogen are in a high structural homogeniety, the absorption procedure weakens even the target reactivity of the lipoprotein (a). This method, therefore, is not applicable to an immuno-turbidimetric quantitative analysis in which an antibody exhibiting a strong reactivity is required for the measurement of the subject compounds within a comparatively short period of time. Even if applied, it provides only an extremely low sensitivity. It was impossible to determine a lipoprotein (a) content of such a low level as that occurring in human blood.

Development of a highly reproducible analytical method of lipoprotein (a) which can give a constantly accurate measurement by using a highly specific monoclonal antibody and does not exhibit a cross-reactivity with plasminogen is therefore desired.

However, a problem in monoclonal antibodies is their capability of recognizing specifically only one type of antigen-determinant site of an antigen with which it reacts Because of this, the antigen-antibody product obtained by the reaction is a product from one antibody molecule and two antigen molecules, even if the reaction is completed. Thus, such a product is extremely small as compared with products obtained from polyclonal antibodies, which are complexes of antibodies and capable of recognizing a number of various antigen-determinant sites, and antigens reactive with such polyclonal antibodies. Products obtained from an antigen and an monoclonal antibody therefore cannot be detected by normal immuno-turbidimetric measurement. Thus, applying the immuno-turbidimetric analysis to one type of monoclonal antibody is impossible.

It is thus necessary for the immuno-turbidimetry using monoclonal antibody to use two or more types of monoclonal antibodies which recognize different antigen-determinant sites in admixture. However, as mentioned above, since apoprotein (a) possesses a structure very similar to plasminogen, specific antigen-determinant sites is considered to be extremely limited, it is very difficult to obtain plurality of specific monoclonal antibodies which cooperate with each other for the formation of macomolecular antigen-antibody bound material. Because of these reasons, the measurement of human lipoprotein (a) by immunoturbidimetric analysis using monoclonal antibodies has been considered very difficult. No monoclonal antibodies usable in such an immuno-turbidimetric analytical method have been known in the art.

In view of this situation, the present inventors have prepared a large number of monoclonal antibodies reactive with human lipoprotein (a) to study their characteristics not only by conventional enzymatic immune methods but also by the immuno-turbidimetric method. As a result, the present inventors discovered several monoclonal antibodies which individually react with human lipoprotein (a) in serum with specificity to produce bound products which can be well detected by a conventional spectrophotometer. Such a finding has led to the completion of the present invention.

## SUMMARY OF THE INVENTION

Accordingly, an object of the present invention is to provide monoclonal antibodies which are reactive with human lipoprotein (a) and do not exhibit an immune cross-reactivity with human plasminogen and apoprotein B.

Another object of the present invention is to provide a method of measuring human lipoprotein (a) by an immunoturbidimetric analysis in which at least one of said monoclonal antibody is used.

Other objects, features and advantages of the invention will hereinafter become more readily apparent from the following description.

## BRIEF DESCRIPTION OF THE DRAWINGS

Figure 1 is a drawing showing reactivities of monoclonal antibody No. 28205 against apoprotein (a), apoprotein B, and human plasminogen by the Western Blotting method.

Figure 2 is a calibration curve used for the measurement of human lipoprotein (a) by using monoclonal antibody No. 28205.

## DETAILED DESCRIPTION OF THE INVENTION AND PREFERRED EMBODIMENTS

Monoclonal antibodies of the present invention are characterized by their specific reactivity with human lipoprotein (a); that is, they are reactive with human lipoprotein (a), but do not exhibit an immune cross-reactivity with human plasminogen and apoprotein B. Such monoclonal antibodies include antibodies of the type which either alone or in combination produce precipitate or turbidity with human lipoprotein (a).

Monoclonal antibodies of the present invention can be prepared according to a conventional method by using human lipoprotein (a) as an immune antigen. Human lipoprotein (a) used in the preparation of the monoclonal antibodies as an immune antigen is a lipoprotein separated and purified by the present inventors from human serum. In the preparation of antibodies of the present invention, lipoprotein (a) is not necessarily purified. It is possible to use a crude product containing the lipoprotein (a). In a specific preferable example of the preparation of antibody of the present invention, transformed mammal cells immuned by lipoprotein (a) as an immune antigen are fused with mammal plasmacytoma to produce hybridomas. A clone recognizing the lipoprotein (a) is selected from the hybridomas. The target monoclonal antibody is then obtained from the clone.

In the above process, there are no limitations to the mammal cells to be transformed with the immune antigen, i.e., the lipoprotein (a). It is desired that the immune antigen be selected taking its compatibility with the mammal plasmacytoma to be fused into consideration. Mice, rats, and the like are generally preferably used.

The immunization is carried out according to a conventional method, e.g., by intravenously or intraperitoneally injecting the lipoprotein (a) into the animal. More specifically, lipoprotein (a) is diluted with PBS or physiological saline to a suitable concentration, and injected to the animal, together with a suitable adjuvant, if necessary, several times at an interval of 2-21 days until the total amount reaches 1-100 μl/animal. A conventional carrier may be used for the injection. Spleen cells enucleated from the animal 3 days after the completion of the injection of lipoprotein (a) is desirable for use as immune cells.

Various known myeloma cells can be used as mammal plasmacytoma to be fused with the above immune cells. Such myeloma cells include, for example, p3 (p3/x63-Ag8) [Nature, 256, 495-497 (1975)], p3-U1 [Current Topics of Microbiology and Immunology, 81, 1-7 (1987)], NS-1 [Eur, J. Immunol, 6, 511-519 (1976)], MPC-11 [Cell, 8, 405-415 (1976)], PS2/0 [Nature, 276, 269-270 (1978)], FO [J. Immunol., Meth., 35, 1-21 (1980)], x63, 6, 5, 3 [J. Immunol., 123, 1548-1550 (1979)], S194 [J. Exp. Med. 148, 313-323 (1978)], and R210 [Nature, 277, 131-133 (1979)] of rat, and the like

The fusion of the immune cell and the plasmacytoma can be carried out basically in accordance with known methods, for example, by the method of Milestein et al [Methods Enzymol., 73, 3-46 (1981)], in the presence of a fusion accelerator and in a conventional nutritious medium. Conventional fusion accelerators, e.g., polyethylene glycol (PEG), sendai virus (HVJ), etc., can be used, optionally with the addition of adjuvants such as dimethylsulfoxide and the like in order to promote the efficiency of the fusion. A fusion ratio of the immune cells and the plasmacytoma may be the conventional ones, e.g., about 1-10 immune cells per one plasmacytoma. As a medium for the fusion, any medium used for the cultivation of the plasmacytoma, e.g., PRMI 1640 medium, MEM medium, as well as other various media used for the cultivation of this type of cells can be used. Serum obtained by removing serum complement from fatal calf serum (FCS) is a typical example of the medium. The fusion is carried out by thoroughly mixing a prescribed amount of the immune cells and the plasmacytoma and blending this mixture with a medium to which about 30-60% (w/v) of a PEG (e.g., PEG with an average molecular weight of 1,000-6,000) solution which has been heated to about 37°C in advancemis added. The cultivation in the HAT medium is continued for a period sufficient for cells other than hybridoma (e.g., unfused cells) to die, usually for several days to several weeks. Hybridoma thus obtained is subjected to a conventional limiting dilution method to detect the target cell line producing the antibody and to monocloning.

The detection of the antibody-producing cell lines is carried out according to a standard method commonly used for the detection of antibodies [Hybridoma and Monoclonal Antibody, R&D Planning Co., 30-53, (1982)], such as, for example, the ELISA method [Engvall, E. Methods Enzymol., 70, 419-439 (9180)], the plaque method, the spot method, the agglomeration reaction method, the Ouchterlony method, the radio immunoassay (RIA), and the like. Use of the above lipoprotein (a) as an antigen for the detection is desirable.

The hybridoma producing the target monoclonal antibody thus obtained can be cultivated over generations in conventional media and can be stored in liquid nitrogen for a long period of time.

Collection of monoclonal antibody of the present invention from the hybridoma can be performed by cultivating the hybridoma according to a conventional method and obtaining the monoclonal antibody as a supernatant or by administering the hybridoma to a mammal with which the hybridoma is compatible to proliferate and by collecting it from the ascites fluid. The former method is adaptable to the production of high purity monoclonal antibody and the latter to massproduction of the monoclonal antibody.

The monoclonal antibody thus obtained may be purified by means of salting, gel filtration, affinity chromatography, or the like.

Human lipoprotein (a) can be analyzed at a high sensitivity and precision and with a high specificity in a simple manner by the use of the monoclonal antibody by a conventional immunoassay, such as the enzymatic immunoassay (EIA), the rocket method, the immuno-turbidimetry, or the like. A particularly preferable method is the immunoturbidimetry because of its adaptability to automatic analysis, enabling a large number of samples to be measured at one time. Specifically, an amount of lipoprotein (a) in a sample such as human plasma, human serum, or the like can be determined by adding one or more of the monoclonal antibodies of the present invention to the sample for the reaction and by measuring changes in the absorbance before and after the reaction.

Establishing the measurement system and modification and application of such a system can be made according to manners known in the art.

Because the monoclonal antibodies of the present invention react with human lipoprotein (a) with specificity and do not exhibit an immunological cross-reactivity with plasminogen and apoprotein B, they are useful for the determination of human lipoprotein (a) in clinical samples by immunoassay, particularly by immuno-turbidimetry, thus enabling to screen various heart diseases and to diagnose the status of the diseases.

Other features of the invention will become apparent in the course of the following description of the exemplary embodiments which are given for illustration of the invention and are not intended to be limiting thereof.


EXAMPLES


Example 1 <Preparation of Antigen>

Lyophillized serum stored at -20°C until the biginning of the experiment was dissolved at room temperature to separate fractions containing a large amount of lipoprotein (a) (density: 1.06-1.12 kg/l) by ultracentrifugation. The fractions were applied to a 100x2.6 cm sephallose CL-6B column and eluted at 4°C with a 0.1 M Tris buffer containing 0.15 M NaCl (pH 7.4) at a rate of 8 ml/hour to collect fractions, 5 ml each. Since high molecular compounds are eluted first, 280 nm absorption under these conditions indicated peaks in the order of lipoprotein (a), LDL (low density lipoprotein), and HDL (high density lipoprotein). Each fraction was analyzed by the polyacrylamide electrophoresis to collect fractions exhibiting only the first peak corresponding to lipoprotein (a), thus avoiding contamination of LDL. The fractions thus obtained was served as antigen solutions. Example 2 <Preparation of Anti-human Lipoprotein (a)>

The protein concentration of the antigen solution obtained in Example 1 was determined by the Lawry method using a cattle albumin solution as a standard. A solution corresponding to 10 μg of the protein concentration was thoroughly blended with a 100 μl complete Freund's adjuvant and intraperitoneally injected into a

EP 0 506 523 A2

BALB/c mouse. Meanwhile, as a booster immune, the above solution of 10 µg of the protein concentration was thoroughly blended with an incomplete Freund's adjuvant and intraperitoneally injected twice at an interval of two weeks since the first injection. After one week following the final injection, 10 µl of the antigen solution was mixed with physiological saline and injected through the caudal vein. Three days thereafter, the spleen was extracted, well loosened, and washed with a medium (PRMI 1640). The spleen cells ($2.5 \times 10^8$) thus prepared were mixed with myeloma cells ($2.5 \times 10^7$) of a SP2/0 Ag14 mouse, which were thoroughly washed with the medium in the same manner. The mixture (0.25 ml) was slowly dropped into a medium (50 w/v% PEG). After blending the mixture for 1 minute, PEG was diluted by slowly adding 8 ml of GKN medium to terminate the reaction. The resulting mixture was centrifuged for 5 minutes at 1,500 rpm to collect cells. The cells were washed with 2 ml of the medium and suspended into 30 ml of HAT medium. 0.1 ml of portions of the suspension was inoculated into each well of a 96-hole microplate, and incubated at 37°C in 8% $CO_2$ for 10 days. The supernatant of each well was replaced with 0.2 ml of HT medium (the same medium as HAT medium, but not containing aminopterin). This procedure was repeated 3 times and the characteristics of the antibody contained in the supernatant finally obtained were determined. In such determination, the antigen solution containing lipoprotein (a) used for the immunization was suitably diluted and charged into each microplate to absorb it to the surface of the wells. The above supernatant was added and incubated, followed by the addition of anti-mouse IgG labeled with peroxidase to determine the antibody activity using the peroxidase activity remaining in the well as a standard. Furthermore, LDL obtained by a conventional method from plasminogen and human serum was charged into the microplate to absorb it to its surface to determine the antibody activity against each component in the same way as employed for the above-mentioned determination of the antibody activity against human lipoprotein (a). Through these procedures, wells reacting only with human lipoprotein (a) were selected. The cloning by the limiting dilution method and the antibody activity determination were repeated twice on the culture broth of the selected wells to finally obtain 6 clones producing antibodies exhibiting specificity toward human lipoprotein (a). The clones were then intraperitoneally injected to a BALB/c mouse which was treated with pristane. After 10-20 days, ascites fluid of the mouse was collected several times to obtain monoclonal antibodies therefrom. The classes and subclasses were determined on the monoclonal antibodies. The results are shown in Table 1. Crude IgG fractions were obtained from the ascites fluid by the ammonium sulfate fractionation and ion-exchange chromatography using DEAE sephallose. Feasibility of immuno-turbidimetric quantitative analysis was evaluated on each fraction as follows. 20 µl of serum containing 50 mg/dl of human lipoprotein (a) was blended with 300 µl of 10 mM phosphate buffer (pH 7.0) containing 3% PEG, 0.15 M NaCl, and 3% dextran. The blend was incubated for 5 minutes to measure the absorbance at 340 nm. To the blend was further added 150 µl of crude IgG fraction which was adjusted to 1 mg/ml, and incubated for 5 minutes to measure the absorbance at 340 nm. This absorbance finally obtained was subtracted by the absorbance before the addition of the IgG fraction corrected by the amount of the reaction mixture, thus calculating the increase in the absorbance created by the reaction with human lipoprotein (a). The results are shown in Table 2, in which are shown several clones which exhibited a characteristic increase in the absorbance by the reaction with serum samples containing human lipoprotein (a). Of these, clone No. 28205 monoclonal antibody was deposited with Fermentation Research Institute, Agency of Industrial Science and Technology (FERM P-12114).

TABLE 1

| Antibody No. | Class | Type |
|---|---|---|
| 28203 | G | *Gamma,* K |
| 28204 | G | *Gamma,* K |
| 28205 | G | *Gamma,* K |
| 28206 | G | *Gamma,* K |
| 28207 | G | *Gamma,* K |
| 28209 | G | *Gamma,* K |

5

## TABLE 2

| Antibody No. | Absorbance |
|---|---|
| 28203 | 0.020 |
| 28204 | 0.007 |
| 28205 | 0.053 |
| 28206 | 0.019 |
| 28207 | 0.007 |
| 28209 | 0.004 |

Example 3 <Confirmation of Specificity of Monoclonal Antibody Against Apoprotein (a)>

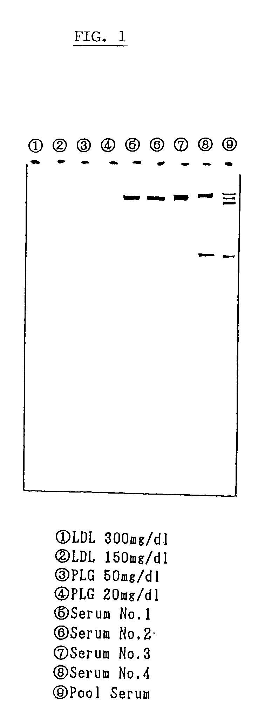

In order to confirm the specificity of monoclonal antibodies against apoprotein (a), the reactivities of monoclonal antibodies obtained by the screening against apoprotein (a), apoprotein B, and human plasminogen were studied by the Western Blotting method. Serum containing human lipoprotein (a) modified with SDS in the presence of 1 mM mercaptoethanol, purified LDL modified with SDS, and commercially available plasminogen were electrophorated with 4-20% SDS gradient polyacrylamide gel using apoprotein (a), apoprotein B, and plasminogen, as test samples. After the electrophoresis, proteins in gels were transcripted onto a nitrocellulose membrane by using a transcripter under the conditions of 20 V for 8 hours. After blocking by immersing it in 1% skimmed milk, the membrane was washed and immersed into an antibody solution adjusted to about 14.2 mg/dl to effect the antigen-antibody reaction. After thoroughly washing the membrane, an anti-mouse IgG antibody solution labeled with peroxidase was charged as a secondary antibody After the reaction, 3,3'-diaminobenzidine was added as a substrate to develop a color. The results are shown in Figure 1, which indicates that No. 28205 reacts only with the protein having a molecular weight corresponding to apoprotein (a) on the lane on which the serum containing human lipoprotein (a) was poured, while no color band was developed on the lanes to which LDL or plasminogen was poured, demonstrating that antibody No. 28205 exhibits a specific antibody activity against apoprotein (a).

Example 4 <Confirmation of Quantitative Analysis>

A serum sample to be tested in which human lipoprotein (a) was superlative was diluted by physiological saline to prepare a series of diluted samples. 20 μl of each diluted sample was mixed with 350 μl of 10 mM phosphate buffer (pH 7.0) containing 3% PEG, 0.15 M NaCl, and 3% dextran, and the mixture was incubated for 5 minutes to measure absorbance at 340 nm. After the addition of 50 μl of a crude IgG solution containing monoclonal antibody of the present invention prepared in Example 2 at a concentration of 0.6 mg/dl, the mixture was incubated for 5 minutes to measure absorbance at 340 nm. This absorbance finally obtained was subtracted by the absorbance before the addition of the IgG fraction corrected by the amount of the reaction mixture to determine absorbance corresponding to each concentration of human lipoprotein (a). The absorbances were plotted against concentrations of human lipoprotein (a) in theoretical samples to obtain a good calibration curve.

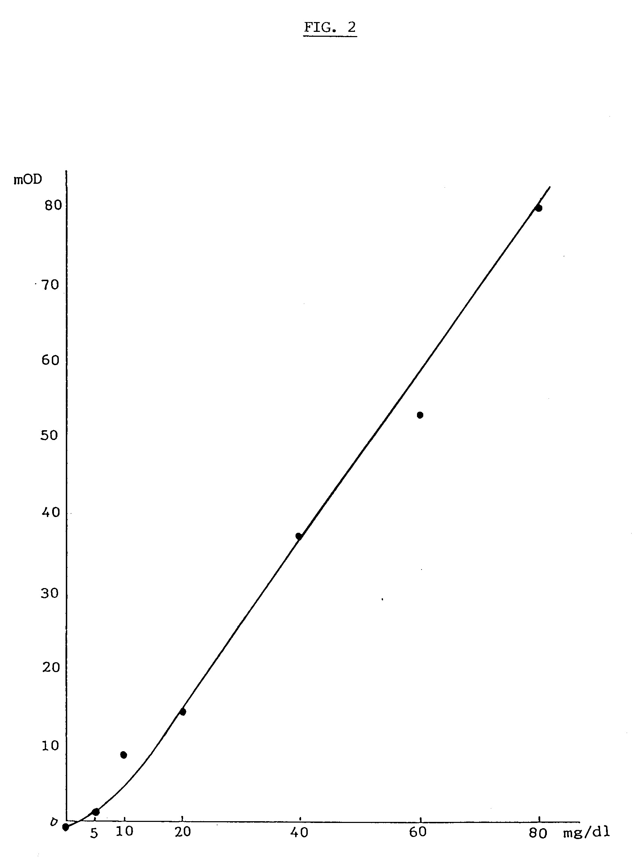

The calibration curve is shown in Figure 2, which demonstrates that the monoclonal antibodies of the present invention can be used individually in the immunoturbidimetric analysis of human lipoprotein (a).

Obviously, numerous modifications and variations of the present invention are possible in light of the above teachings. It is therefore to be understood that within the scope of the appended claims, the invention may be practiced otherwise than as specifically described herein.

## Claims

1. Monoclonal antibodies which are reactive with human lipoprotein (a) and do not exhibit an immune cross-reactivity with human plasminogen and apoprotein B.

2. The monoclonal antibodies according to Claim 1, which can be used individually or in combination in the immuno-turbidimetric quantitative analysis of human lipoprotein (a).

3. A method of measuring human lipoprotein (a) characterized by reacting a sample with at least one monoclonal antibody which is reactive with human lipoprotein (a) and do not exhibit an immune cross-reactivity with human plasminogen and apoprotein B and measuring the absorbance of the resulting reaction mixt-ure.

FIG. 1

① ② ③ ④ ⑤ ⑥ ⑦ ⑧ ⑨

①LDL 300mg/dl
②LDL 150mg/dl
③PLG 50mg/dl
④PLG 20mg/dl
⑤Serum No.1
⑥Serum No.2
⑦Serum No.3
⑧Serum No.4
⑨Pool Serum

FIG. 2